(19) Europäisches Patentamt
European Patent Office
Office européen des brevets

(11) Publication number: **0 167 154 B1**

# (12) EUROPEAN PATENT SPECIFICATION

(45) Date of publication of patent specification: **03.01.90**

(51) Int. Cl.[5]: **C 07 D 205/08,** C 07 F 7/10, C 07 F 7/18

(21) Application number: **85108208.1**

(22) Date of filing: **03.07.85**

## (54) Process for preparing 4-acetoxy-3-hydroxyethylazetizin-2-one derivatives.

(30) Priority: **05.07.84 JP 139797/84**
**14.01.85 JP 4724/85**

(43) Date of publication of application:
**08.01.86 Bulletin 86/02**

(45) Publication of the grant of the patent:
**03.01.90 Bulletin 90/01**

(84) Designated Contracting States:
**BE CH DE FR GB IT LI NL**

(56) References cited:

**CHEMICAL & PHARMACEUTICAL BULLETIN, vol. 29, no. 10, October 1981, Pharmaceutical Society of Japan; YOSHIDA et al.: "2-(Alkylthio)-penem-3-carboxylic acids. IV. Synthesis of (Hydroxyethyl)-azetidinone precursors to 1-Thia Analogs of Thienamycin", pp. 2899-2909**

**TETRAHEDRON, vol. 39, no. 13, 1983, Oxford, SHIOZAKI et al.: "Stereocontrolled syntheses of chiral and racemic key intermediates to Thienamycin from D-Allo-Threonine and Trans-Crotonic acid", pp. 2399-2407**

(73) Proprietor: **KANEGAFUCHI KAGAKU KOGYO KABUSHIKI KAISHA**
**2-4 Nakanoshima 3-chome**
**Kita-ku Osaka-shi Osaka-fu (JP)**

(72) Inventor: **Ohashi, Takehisa**
**9-14, Obanoyama-cho 3-chome Shinohara**
**Nada-ku Kobe-shi Hyogo-ken (JP)**
Inventor: **Kazunori, Kan**
**9-30-616, Maikodai 2-chome Tarumi-ku**
**Kobe-shi Hyogo-ken (JP)**
Inventor: **Sada, Isao**
**1459-1, Fujie**
**Akashi-shi Hyogo-ken (JP)**
Inventor: **Miyama, Akimasa**
**3-6-24, Okihama-cho Takasago-cho**
**Takasago-shi Hyogo-ken (JP)**
Inventor: **Watanabe, Kiyoshi**
**15-41, Matsugaoka 5-chome**
**Akashi-shi Hyogo-ken (JP)**

(74) Representative: **Türk, Gille, Hrabal**
**Bruckner Strasse 20**
**D-4000 Düsseldorf 13 (DE)**

**EP 0 167 154 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a process for preparing 4-acetoxy-3-hydroxyethylazetizin-2-one which has hydroxyethyl group, wherein hydroxy group is protected, at C-3-position and acetoxy group at C-4-position. It is known that 4-acetoxy-3-hydroxyethylazetizin-2-one derivatives are the useful intermediates for preparing carbapenem β-lactam antibiotics such as thienamycin and penem β-lactam antibiotics (cf., for example, Tetrahedronletters by Laider et al., vol. 23, page 2293, 1982 and Chem. Pharm. Bull. by Yoshida et al., vol. 29, page 2899, 1981).

Hitherto, processes for synthesizing 4-acetoxy-3-hydroxyethylazetizin-2-one derivatives have been known such as synthesis from 6-aminopenicillanic acid (cf. Chem. Pharm. Bull. by Yoshida et al., vol. 29, page 2899, 1981), synthesis from threonine (cf. Tetrahedron by Shiozaki et al, vol. 39, page 2399, 1983), synthesis from aspartic acid (cf. Tetrahedronletters by Laider et al., vol. 23, page 2293, 1982), and the like. However, these processes have a difficult point that industrially unfavourable heavy metals such as mercury acetate and lead tetraacetate are employed in order to introduce acetoxy group at C-4-position of the β-lactam ring. The inventors have found a process for preparing a novel β-lactam compound having O-protected hydroxyethyl group at C-3-position and silylether group at C-4-position by the reaction of enolsilylether with chlorosulfonylisocyanate and this process has already been made patent application. The inventors further found that 4-acetoxy-3-hydroxyethylazetizin-2-one could be easily prepared by using the above-mentioned β-lactam compound and thus the present invention was completed.

According to the present invention, there is provided a process for preparing 4-acetoxy-3-hydroxyethylazetizin-2-one having the general formula (II):

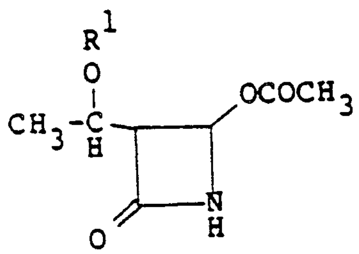

(II)

wherein $R^1$ is a protective group for hydroxy group, where the β-lactam compound having the general formula (I):

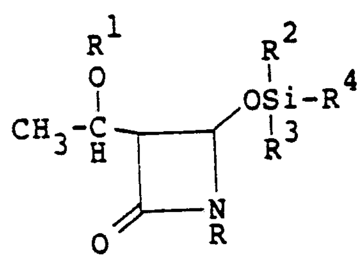

(I)

wherein $R^1$ is as defined above, and $R^2$, $R^3$ and $R^4$ are a lower alkyl group of $C_1$ to $C_4$, phenyl group or an aralkyl group and R is a protective group for N, with acetic anhydride in an organic solvent in the presence of a base, at a temperature ranging from −50°C to room temperature, and removing the protective group for N, the amount of acetic anhydride being from 1 to 20 times moles of said β-lactam compound (I), the amount of said base being from 1 to 5 times moles of said β-lactam compound (I), and said base being dimethylaminopyridine or dimethylaminopyridine in combination with pyridine, picoline or lutidine.

The β-Lactam compound having the general formula (I) is prepared by introducing substituent group R at N of the β-lactam compound having the general formula (I'):

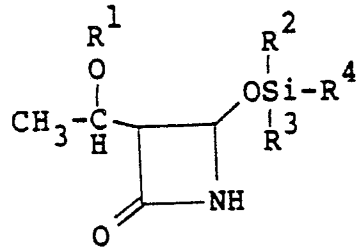

(I')

wherein $R^1$, $R^2$, $R^3$ and $R^4$ are as above. As is shown in the application of the inventors (EP—A—167155), the

β-lactam compound having silylether group at C-4-position and having the general formula (I') can be easily obtained by the process of the following reaction scheme:

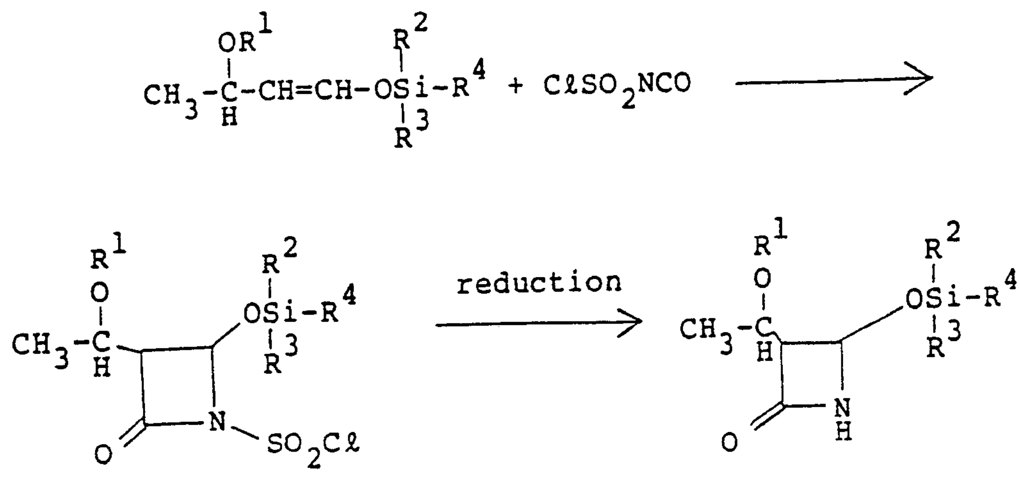


(I')

The β-lactam compound having a protective group for N and having the general formula (I) can be obtained by the reaction of the β-lactam compound having the general formula (I') and the reagent having the general formula of R—X, wherein R is as above and X is a halogen. The examples of the reagent having the general formula of R—X are, for instance, trialkyl-silyl halide such as t-butyldimethylsilyl chloride, isopropyldimethylsilyl chloride, isobutyldimethylsilyl chloride, trimethylsilyl chloride, and the like, alkyloxyoxalyl chloride, aralkyloxyoxalyl chloride or allyloxyoxalyl chloride such as

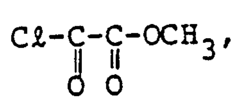, 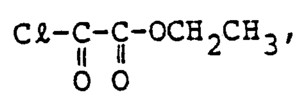, 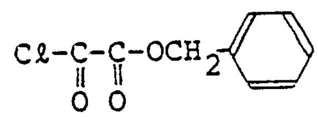,

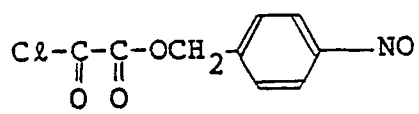, 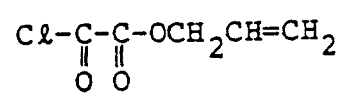,

and the like. t-Butyldimethylsilyl chloride is particularly preferable and t-butyldimethylsilyl group can be introduced as a protecting group.

The compound having the general formula (I) can be obtained by conducting an elimination of hydrogen chloride in the reaction of the β-lactam compound (I') with the reagent having the general formula of R—X in the solvent such as DMF or methylene chloride in the presence of base such as triethylamine and imidazole, followed by distilling away a solvent, extracting and distilling away the extraction solvent.

The examples of the O-protective group of $R^1$ for hydroxy group at C-3-position of the β-lactam compound having the general formula (I) are, for instance, trialkylsilyl group having the general formula (III):

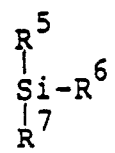

(III)

wherein $R^5$, $R^6$ and $R^7$ are a lower alkyl group of $C_1$ to $C_4$ such as tert-butyldimethylsilyl group, triisopropylsilyl group, isopropyldimethylsilyl group, and isobutyldimethylsilyl group, t-butyl group, benzyl group, trichloroethoxycarbonyl group, tert-butoxycarbonyl group, p-nitrobenzyloxycarbonyl group and the like. tert-Butyldimethylsilyl group or isopropyldimethylsilyl group, which are stable during the reaction and can be selectively deprotected by acid treatment, are particularly preferable. Though $R^2$, $R^3$ and $R^4$ of the β-lactam compound having the general formula (I) can be the same or different from each other and selected from a lower alkyl group of $C_1$ to $C_4$ such as methyl, ethyl, isopropyl, isobutyl and tert-butyl group, phenyl

group, benzyl group or aralkyl group such as p-nitrobenzyl group, preferably all of $R^2$, $R^3$ and $R^4$ are methyl group.

Base, solvent and reaction temperature are the factors which affect the yield when the β-lactam compound prepared as mentioned above and having the general formula (I):

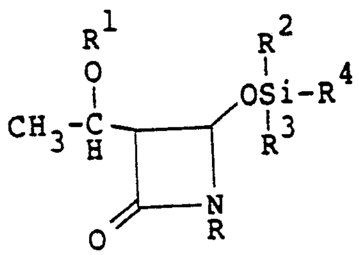

(I)

wherein $R^1$, $R^2$, $R^3$, $R^4$ and R are as above, is reacted with acetic anhydride in the organic solvent in the presence of base to convert the said β-lactam compound into the desired 4-acetoxy-3-hydroxyethylazetizin-2-one derivative having the general formula (II'):

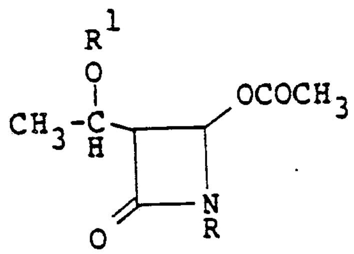

(II')

wherein $R^1$ and R are as above. Halogenated hydrocarbon or aromatic hydrocarbon, preferably halogenated hydrocarbon such as methylene chloride can be employed as a reaction solvent. The base to be used is dimethylaminopyridine, which can also be used in combination with pyridine, lutidine or picoline. The amount of the base employed is 1 to 5 times moles, preferably 1 to 3 times moles of the β-lactam compound having the general formula (I) and the amount of acetic anhydride employed as the reagent for introducing acetoxy is 1 to 20 times moles, preferably 3 to 15 times moles of the β-lactam compound having the general formula (I).

The reaction is carried out in the organic solvent such as methylene chloride by adding dimethylaminopyridine and acetic anhydride to the β-lactam compound having the general formula (I) and stirring the mixture at a temperature of −50°C to room temperature, preferably at a temperature of −30 to 5°C until the compound having the general formula (I) disappears. When pyridine or picoline is added in addition to dimethylaminopyridine, the yield can be improved. After the reaction is completed, extraction solvent such as methylene chloride or hexane is added to the reaction mixture, followed by addition of aqueous sodium bicarbonate, separation of the organic layer, washing the organic layer, dehydration and distilling away the organic solvent to give N-protected β-lactam compound having acetoxy group at C-4-position. The obtained compound having the general formula (II') can be subjected to the next reaction where N-protective group is removed. However, if desired, this compound can be purified by silica-gel column-chromatography.

When N-protective group is removed, it is required that only N-protective group is selectively removed without removal of O-protective group of hydroxyethyl at C-3-position. For example, tetrabutylammonium fluoride is preferably used in case that N-protective group is alkylsilyl group such as tert-butyldimethylsilyl group. A combination of quaternary ammonium halide such as, for example, tetrabutylammonium chloride, tetrabutylammonium bromide, tetramethylammonium chloride, trimethylbenzylammonium chloride with potassium fluoride can provide an equivalent effect to tetrabutylammonium fluoride, and also potassium fluoride alone can be available when methanol is used as the solvent.

The solvent used in the elimination reaction is preferably tetrahydrofuran, acetonitrile or methylene chloride and the reaction is smoothly proceeded by stirring at room temperature. Employing acetic acid in the removal of the protective group results in the improvement of the yield. After the reaction is completed, the extraction solvent such as ethyl acetate is added to the reaction mixture, followed by the addition of the dilute alkali solution. Then, the organic layer is extracted, washed with water, dehydrated and dried, and finally the organic solvent is distilled away to give the desired compound of 4-acetoxy-3-hydroxyethylazetizin-2-one derivatives. 4-Acetoxy-3-hydroxyethylazetizin-2-one derivatives can be purified by crystallization from n-hexane or petroleum ether or silica-gel column-chromatography.

The present invention is more particularly explained by the following non-limiting examples.

Example 1

[Preparation of (3R,4R)-4-acetoxy-3-[(R)-1-tert-butyldimethylsilyloxyethyl]-azetizin-2-one]

There was dissolved 1.0 g of (3R,5R)-3-(1-tert-butyldimethylsilyloxyethyl)-4-trimethylsilyloxyazetizin-2-one in 10 ml of DMF, to which 0.89 g of triethylamine and 0.61 g of tert-butyldimethylsilyl chloride were added and the mixture was stirred for 9 hours at room temperature. After the reaction was completed, DMF was distilled away under reduced pressure and thereto 30 ml of hexane was added. After the solution was washed successively with 2.5% aqueous solution of $NaHCO_3$, aqueous solution of hydrochloric acid of pH 3 and saturated solution of salt and dried with magnesium sulfate, the solvent was distilled away to give 1.24 g of the liquid of the crude product.

To 5 ml of methylene chloride was added 1.0 g of the liquid, thereto further 0.85 g of dimethylaminopyridine and 1.1 ml of acetic anhydride was added and the mixture was reacted for 6 hours at room temperature. After the reaction mixture was washed successively with 5% aqueous solution of $NaHCO_3$, aqueous solution of hydrochloric acid of pH 3 and saturated solution of salt and dried with magnesium sulfate, the solvent was distilled away to give 0.8 g of the liquid of the crude product. The liquid was purified by a silica-gel column-chromatography (benzene : hexane = 2 : 1) to give 0.5 g of (3R,4R,5R)-4-acetoxy-1-(tert-butyldimethylsilyl)-3-(1-tert-butyldimethylsilyloxyethyl)-azetizin-2-one as a liquid.

There was added 0.5 g of the liquid to 2 ml of THF, to which 0.4 g of tetrabutylammonium fluoride and 0.17 g of acetic acid in 2 ml of THF solution was added and the mixture was stirred for 30 minutes at room temperature. After adding 20 ml of ethyl acetate to the reaction mixture, the resultant was washed successively with 5% aqueous solution of $NaHCO_3$ and saturated solution of salt and dried with magnesium sulfate. The solvent was distilled away to give 0.30 g of the desired compound as a crystal. The obtained crystal was purified by a silica-gel column-chromatography (benzene : ethyl acetate = 6 : 1) to give 0.27 g of the desired β-lactam as a solid.

mp: 107° to 108°C

$[\alpha]_D^{25} = +50°$ ($C=0.5$ $CHCl_3$)

$^1$HNMR (90 MHz, $CDCl_3$)δ, (ppm)

0.08 (6H, s), 0.84 (9H, s), 1.20 (3H, d), 2.01 (3H, s), 3.04 (1H, dd), 4.12 (1H, m), 5.76 (1H, d) and 6.73 (NH)

Example 2

[Preparation of (3R,4R)-4-acetoxy-3-[(R)-1-tert-butyldimethylsilyloxyethyl]-azetizin-2-one]

There was dissolved 1 g of (3R,4R)-1-tert-butyldimethylsilyl-3-[(R)-1-tert-butyldimethylsilyloxyethyl]-4-trimethylsilyloxyazetizin-2-one into 10 ml of methylene chloride, thereto 0.85 g of dimethylaminopyridine and 0.71 g of acetic anhydride were added and the mixture was reacted for one day at 0°C. After the reaction mixture was diluted with hexane and washed successively with 5% aqueous solution of $NaHCO_3$, aqueous solution of hydrochloric acid of pH 4 and saturated solution of salt, the resultant was dried with magnesium sulfate and the solvent was distilled away to give 0.85 of the liquid of the crude product. The obtained liquid was purified by a silica-gel column-chromatography (hexane : ether = 30 : 1) to give 0.40 g of (3R,4R)-4-acetoxy-1-tert-butyldimethylsilyl)-3-[(R)-1-tert-butyldimethylsilyloxyethyl)azetizin-2-one as a colorless liquid.

There was added 2 ml of THF to the obtained liquid, thereto 0.26 of tetrabutylammonium fluoride and 0.12 g of acetic acid in 2 ml of THF were added and the mixture was stirred for 30 minutes at room temperature. After adding 20 ml of ethyl acetate to the reaction mixture, the resultant was washed successively with 5% aqueous solution of $NaHCO_3$ and saturated solution of salt, and dried with magnesium sulfate. The solvent was distilled away to give 0.29 g of the solid. The obtained solid was recrystallized from hexane to give 0.20 g of the desired β-lactam as colorless needles.

The properties of the obtained β-lactam were agreed with the values shown in Example 1.

Example 3

[Preparation of (3R,4R)-4-acetoxy-3-[(R)-1-tert-butyldimethylsilyloxyethyl]-azetizin-2-one]

There was dissolved 1 g of (3R,4R)-1-tert-butyldimethylsilyl-3-[(R)-1-tert-butyldimethylsilyloxyethyl]-4-trimethylsilyloxyazetizin-2-one into 10 ml of methylene chloride, thereto 0.85 g of dimethylaminopyridine, 0.75 g of 2,6-lutidine and 1.42 g of acetic anhydride were added and the mixture was reacted for 44 hours at −15°C. After the reaction mixture was diluted with ether and washed successively with 5% aqueous solution of $NaHCO_3$, aqueous solution of hydrochloric acid of pH 4 and saturated solution of salt, the resultant was dried with magnesium sulfate. The solvent was distilled away to give 0.88 g of the liquid of the crude product. The obtained liquid was purified by a silica-gel column-chromatography (hexane : ethyl acetate = 100 : 1) to give 0.58 g of (3R,4R)-4-acetoxy-tert-butyldimethylsilyl)-3-[(R)-1-tert-butyldimethylsilyloxyethyl)azetizin-2-one as a liquid.

There was dissolved the obtained liquid into 2 ml of THF, thereto 0.38 g of tetrabutylammonium fluoride and 0.17 g of acetic acid in 2 ml of THF was added and the mixture was stirred for 30 minutes at room temperature. After adding 20 ml of ethyl acetate to the reaction mixture, the resultant was washed successively with 5% aqueous solution of $NaHCO_3$ and saturated solution of salt, and dried with magnesium sulfate. The solvent was distilled away to give 0.42 g of a solid. The obtained solid was purified by a silica-gel column-chromatography (hexane : ether = 10 : 3) to give 0.39 g of the desired β-lactam as colorless needles.

The properties of the obtained β-lactam were agreed with the values shown in Example 1.

Example 4

[Preparation of (3R,4R)-4-acetoxy-3-[(R)-1-isopropyldimethylsilyloxyethyl]-azetizin-2-one]

There was dissolved 1.2 g of 3-[(R)-1-isopropyldimethylsilyloxyethyl]-4-trimethylsilyloxyazetizin-2-one ((3R,4R,5R)-form : (3S,4S,5R)-form = 5 : 1) into 12 ml of DMF, thereto 0.52 g of triethylamine and 0.79 g of tert-butyldimethylsilyl chloride were added and the mixture was stirred for 9 hours at room temperature. After the reaction was completed, DMF was distilled away under reduced pressure and thereto 30 ml of hexane was added. After the reaction mixture was washed successively with 2.5% aqueous solution of $NaHCO_3$, aqueous solution of hydrochloric acid of pH 5 and saturated solution of salt, and was dried with magnesium sulfate, the solvent was distilled away to give 1.13 g of the liquid of the crude product.

There was added 0.90 g of the obtained liquid to 10 ml of methylene chloride, thereto 0.79 g of dimethylaminopyridine and 0.61 ml of acetic anhydride were added and the mixture was reacted for 16 hours at 4°C. After the reaction mixture was washed successively with 5% aqueous solution of $NaHCO_3$, aqueous solution of hydrochloric acid of pH 5 and saturated solution of salt, and dried with magnesium sulfate, the solvent was distilled away to give 0.70 g of the liquid of the crude product. The obtained liquid was purified by a silica-gel-column-chromatography (hexane : ether = 100 : 3) to give 0.20 g of 4-acetoxy-1-tert-butyldimethylsilyl)-3-(1-isopropyldimethylsilyloxyethyl)azetizin-2-one as a liquid.

There was added 0.20 g of the obtained liquid to 2 ml of THF, thereto 0.13 of tetrabutylammonium fluoride and 0.03 g of acetic acid in 2 ml of THF was added and the mixture was stirred for 30 minutes at room temperature. There was added 60 ml of ethyl acetate to the reaction mixture and the mixture was washed successively with 5% aqueous solution of $NaHCO_3$ and saturated solution of salt, and dried with magnesium sulfate. The solvent was distilled away to give 0.14 g of the desired crude product ((3R,4R,5R)-form : (3S,4S,5R)-form = 5 : 1). Recrystallization of the product from hexane gave 0.58 g of (3R,4R,5R)-form as white crystals.

$^1$HNMR (90 MHz, $CDCl_3$) ((3R,4R,5R)-form) δ (ppm):

0.04 (6H, s), 0.90 (7H), 1.23 (3H, d), 2.06 (3H, s), 3.13 (1H, dd), 4.13 (1H, m), 5.76 (1H, d) and 6.40 (NH)

$[\alpha]_D^{25} = +54.2°$ (C=0.5, $CHCl_3$)

mp: 92° to 94°C

**Claims**

1. A process for preparing a 4-acetoxy-3-hydroxyethylazetidin-2-one derivative having the general formula (II):

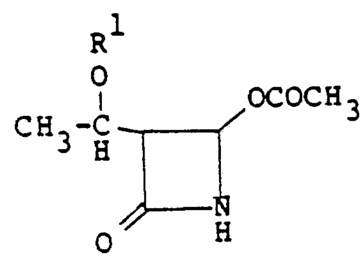

(II)

wherein $R^1$ is a protective group for the hydroxyl group, which comprises reacting a β-lactam compound having the general formula (I):

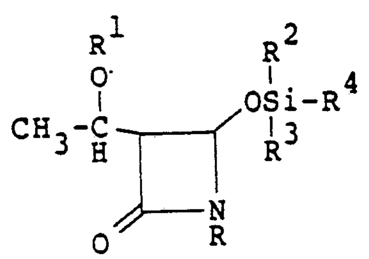

(I)

wherein $R^1$ is as defined above, and $R^2$, $R^3$ and $R^4$ are a lower alkyl group of $C_1$ to $C_4$, phenyl group or an aralkyl group and R is a protective group for N, with acetic anhydride in an organic solvent in the presence of a base, at a temperature ranging from −50°C to room temperature, and removing the protective group for N; the amount of acetic anhydride being from 1 to 20 times moles of said β-lactam compound (I), the amount of said base being from 1 to 5 times moles of said β-lactam compound (I), and said base being dimethylaminopyridine or dimethylaminopyridine in combination with pyridine, picoline or lutidine.

2. The process of Claim 1, wherein $R^1$ is the general formula (III):

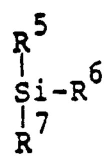

(III)

wherein $R^5$, $R^6$ and $R^7$ are a lower alkyl group of $C_1$ to $C_4$.

3. The process of Claim 1, wherein $R^1$ is t-butylmethylsilyl group.

4. The process of Claim 1, wherein $R^1$ is isopropyldimethylsilyl group.

5. The process of any of Claims 1 to 4, wherein R is t-butylmethylsilyl group.

6. The process of any of Claims 1 to 5, wherein $R^2$, $R^3$ and $R^4$ are methyl groups.

7. The process of any of Claims 1 to 6, wherein the organic solvent is halogenated hydrocarbon.

8. A process for preparing 4-acetoxy-3-hydroxyethylazetidin-2-one having the general formula (II):

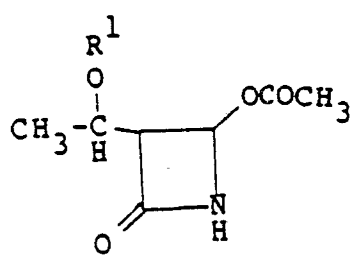

(II)

wherein $R^1$ is a protective group for hydroxy group, which comprises reacting the compound having the general formula (I'):

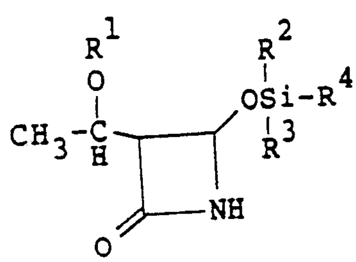

(I')

wherein $R^1$ is as defined above, and $R^2$, $R^3$ and $R^4$ are a lower alkyl group of $C_1$ to $C_4$, phenyl group or an aralkyl group, with a reagent having the formula R—X, wherein R is a protective group for N and X is a halogen atom, to form a compound having the general formula (I):

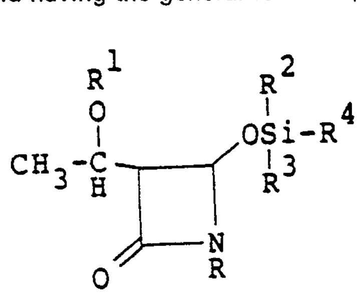

(I)

wherein R, $R^1$, $R^2$, $R^3$ and $R^4$ are as defined above, reacting the compound (I) with acetic anhydride in an organic solvent in the presence of a base at a temperature ranging from −50°C to room temperature, and removing the protective group for N; the amount of acetic anhydride being from 1 to 20 times moles of said β-lactam compound (I), the amount of said base being from 1 to 5 moles of said β-lactam compound (I), and said base being dimethylaminopyridine or dimethylaminopyridine in combination with pyridine, picoline or lutidine.

**Patentansprüche**

1. Verfahren zur Herstellung eines 4-Acetoxy-3-hydroxyethylazetidin-2-on-Derivates mit der allgemeinen Formel (II):

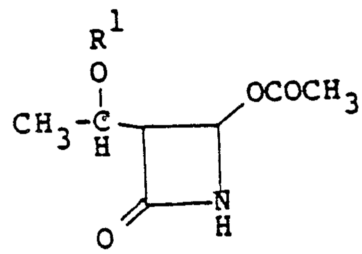

(II)

worin $R^1$ eine Schutzgruppe für die Hydroxylgruppe ist, welches umfaßt Umsetzen einer β-Lactamverbindung mit der allgemeinen Formel (I):

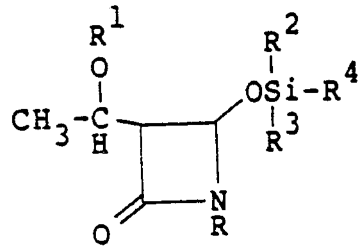

(I)

worin $R^1$ wie oben definiert ist, und $R^2$, $R^3$ und $R^4$ eine nieder-Alkylgruppe von $C_1$ bis $C_4$, Phenylgruppe oder eine Aralkylgruppe sind und R eine Schutzgruppe für N ist, mit Acetanhydrid in einem organischen Lösungsmittel in der Gegenwart einer Base, bei einer Temperatur, die von −50°C bis Raumtemperatur geht, und Entfernen der Schutzgruppe für N; wobei die Menge des Acetanhydrids von 1 bis 20-fache der Mole der β-Lactamverbindung (I) ist, wobei die Menge der Base von 1 bis 5-fache der Mole der β-Lactamverbindung (I) ist und wobei die Base Dimethylaminopyridin oder Dimethylaminopyridin in Kombination mit Pyridin, Picolin oder Lutidin ist.

2. Verfahren nach Anspruch 1, worin $R^1$ die allgemeine Formel (III) hat:

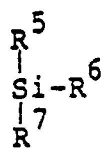

(III)

worin $R^5$, $R^6$ und $R^7$ eine nieder-Alkylgruppe von $C_1$ bis $C_4$ sind.

3. Verfahren nach Anspruch 1, worin $R^1$ t-Butylmethylsilylgruppe ist.

4. Verfahren nach Anspruch 1, worin $R^1$ Isopropyldimethylsilylgruppe ist.

5. Verfahren nach jedem der Ansprüche 1 bis 4, worin R t-butylmethylsilylgruppe ist.

6. Verfahren nach jedem der Ansprüche 1 bis 5, worin $R^2$, $R^3$ und $R^4$ Methylgruppen sind.

7. Verfahren nach jedem der Ansprüche 1 bis 6, worin das organische Lösungsmittel halogenierter Kohlenwasserstoff ist.

8. Verfahren zur Herstellung von 4-Acetoxy-3-hydroxyethylazetidin-2-on mit der allgemeinen Formel (II):

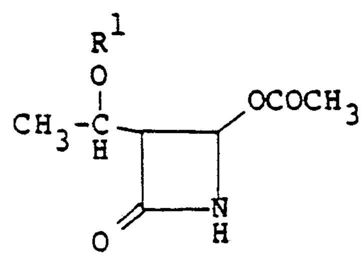

(II)

worin $R^1$ eine Schutzgruppe für Hydroxygruppe ist, welches umfaßt Umsetzen der Verbindung mit der allgemeinen Formel (I'):

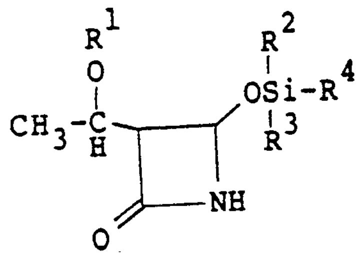

(I')

worin $R^1$ wie oben definiert ist, und $R^2$, $R^3$ und $R^4$ eine nieder-Alkylgruppe mit $C_1$ bis $C_4$, Phenylgruppe oder eine Aralkylgruppe sind, mit einem Reagenz mit der Formel R—X, worin R eine Schutzgruppe für N ist und X ein Halogenatom ist, um eine Verbindung zu bilden mit der allgemeinen Formel (I):

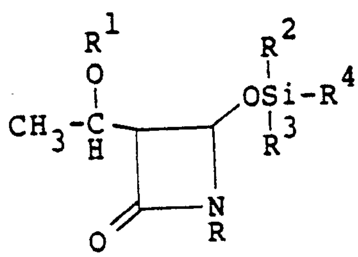

(I)

worin R, $R^1$, $R^2$, $R^3$ und $R^4$ wie oben definiert sind, Umsetzen der Verbindung (I) mit Acetanhydrid in einem organischen Lösungsmittel in der Gegenwart einer Base bei einer Temperatur, die von −50°C bis Raumtemperatur geht, und Entfernen der Schutzgruppe für N; wobei die Menge des Acetanhydrids von 1 bis 20-fache der Mole der β-Lactamverbindung (I) ist, wobei die Menge der Base von 1 bis 5-fache der Mole der β-Lactamverbindung (I) ist, und wobei die Base Dimethylaminopyridin oder Dimethylaminopyridin in Kombination mit Pyridin, Picolin oder Lutidin ist.

**Revendications**

1. Procédé de préparation d'un dérivé de 4-acétoxy-3-hydroxyéthylazétidine-2-one de la formule générale (II):

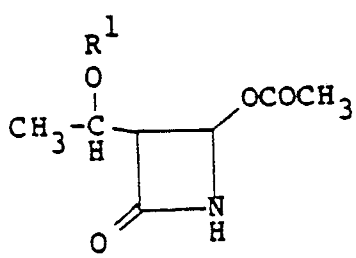

(II)

où $R^1$ est un radical protecteur pour le radical hydroxyle, qui comprend la réaction d'un composé β-lactamique de la formule générale (I):

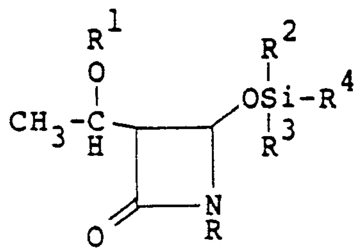

(I)

où $R^1$ est tel que défini ci-dessus et

$R^2$, $R^3$ et $R^4$ sont chacun un radical alcoyle inférieur en $C_1$ à $C_4$, un radical phényle ou un radical aralcoyle et

R est un radical protecteur pour N, avec l'anhydride acétique dans un solvant organique en présence d'une base, à une température s'échelonnant de −50°C à la température ambiante, et l'élimination du

radical protecteur pour N; la quantité d'anhydride acétique étant de 1 à 20 fois le nombre de moles du composé β-lactamique (I), la quantité de base étant de 1 à 5 fois le nombre de moles du composé β-lactamique (I) et la base étant la diméthylaminopyridine ou bien diméthylaminopyridine en combinaison avec la pyridine, la picoline ou la lutidine.

2. Procédé suivant la revendication 1, dans lequel $R^1$ est de la formule générale (III):

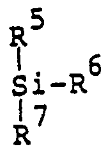

(III)

où $R^5$, $R^6$ et $R^7$ sont chacun un radical alcoyle inférieur en $C_1$ à $C_4$.

3. Procédé suivant la revendication 1, dans lequel $R^1$ est un radical t-butyldiméthylsilyle.

4. Procédé suivant la revendication 1, dans lequel $R^1$ est un radical isopropyldiméthylsilyle.

5. Procédé suivant l'une quelconque des revendications 1 à 4, dans lequel R est un radical t-butyldiméthylsilyle.

6. Procédé suivant l'une quelconque des revendications 1 à 5, dans lequel $R^2$, $R^3$ et $R^4$ sont des radicaux méthyle.

7. Procédé suivant l'une quelconque des revendications 1 à 6, dans lequel le solvant organique est un hydrocarbure halogéné.

8. Procédé de préparation de la 4-acétoxy-3-hydroxyéthylazétidine-2-one de la formule générale (II):

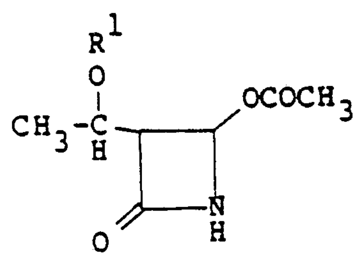

(II)

où $R^1$ est un radical protecteur pour le radical hydroxyle, qui comprend la réaction du composé de la formule générale (I'):

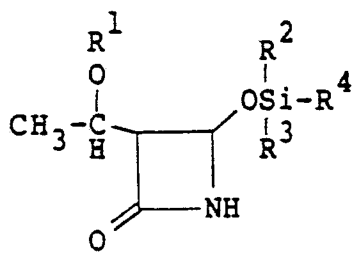

(I')

où $R^1$ est tel que défini ci-dessus, et $R^2$, $R^3$ et $R^4$ sont chacun un radical alcoyle inférieur en $C_1$ à $C_4$, un radical phényle ou un radical aralcoyle avec un réactant de formule

R—X

où R est un radical protecteur pour N et X est un atome d'halogène, pour la formation d'un composé de la formule générale (I):

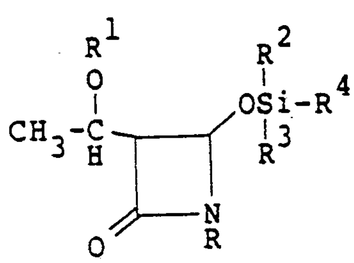

(I)

où R, $R^1$, $R^2$, $R^3$ et $R^4$ sont tels que définis ci-dessus, la réaction du composé (I) avec l'anhydride acétique dans un solvant organique en présence d'une base à une température s'échelonnant de −50°C à la température ambiante, et l'élimination du radical protecteur pour N; la quantité d'anhydride acétique étant de 1 à 20 fois le nombre de moles du composé β-lactamique (I), la quantité de base étant de 1 à 5 fois le nombre de moles du composé β-lactamique (I) et la base étant la diméthylaminopyridine ou bien la diméthylaminopyridine en combinaison avec la pyridine, la picoline ou la lutidine.